(19) Europäisches Patentamt
European Patent Office
Office européen des brevets

(11) Veröffentlichungsnummer: **0 188 216 B1**

# (12) EUROPÄISCHE PATENTSCHRIFT

(45) Veröffentlichungstag der Patentschrift: **15.05.91**

(51) Int. Cl.$^5$: **A61K 7/13**

(21) Anmeldenummer: **86100135.2**

(22) Anmeldetag: **07.01.86**

## (54) Haarfärbemittel-Zubereitung.

(30) Priorität: **12.01.85 DE 3500877**

(43) Veröffentlichungstag der Anmeldung:
**23.07.86 Patentblatt 86/30**

(45) Bekanntmachung des Hinweises auf die Patenterteilung:
**15.05.91 Patentblatt 91/20**

(84) Benannte Vertragsstaaten:
**AT BE CH DE FR GB IT LI NL SE**

(56) Entgegenhaltungen:
**DE-A- 552 268**
**FR-A- 2 434 619**
**GB-A- 2 018 836**

**THE JOURNAL OF THE AMERICAN OIL CHEMISTS' SOCIETY, Band 52, Nr. 7, 1975, Seiten 219-224, New York, US; B.F. WARD, Jr. et al.: "Industrial utilization of C21 Dicarboxylic acid"**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Höffkes, Horst, Dr.**
**Carlo-Schmid-Strasse 113**
**W-4000 Düsseldorf-Hellerhof(DE)**
Erfinder: **Schrader, Dieter**
**Itterstrasse 7**
**W-4000 Düsseldorf 13(DE)**

**EP 0 188 216 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung ist eine flüssige Zubereitung für Oxidationshaarfärbemittel. Solche Zubereitungen bestehen aus Haarfarbstoffvorprodukten und einem kosmetischen Träger, der für die Anwendung auf dem Haar geeignet ist. Als Träger werden bevorzugt entweder cremeförmige Emulsionen vom Typ Wasser-in-Öl oder aber wässrige oder wässrig-alkoholische Lösungen von Seifen verwendet.

Solche Oxidationshaarfärbemittel-Grundlagen auf Basis flüssiger, wässriger oder wässrig-alkoholischer Seifenlösungen zeigen die erwünschte Eigenschaft, nach Zugabe der zur Entwicklung der Oxidationsfarben erforderlichen wässrigen Lösung des Oxidationsmittels ein dickflüssiges bis gelförmiges, gebrauchsfertiges Haarfärbepräparat auszubilden.

Es ist auch bekannt, Haarfärbemittel-Zubereitungen zur Verbesserung der haarkosmetischen Eigenschaften des damit behandelten Haares, insbesondere zur gleichzeitigen Erzeugung einer haaravivierenden Wirkung, mit einem Zusatz an wasserlöslichen kationischen Polymeren zu versehen.

Ein Problem bereitet jedoch die Herstellung flüssiger Zubereitungen für Oxidationshaarfärbemittel mit einem Gehalt an Seifen und kationischen Polymeren, da solche Zubereitungen zur Inhomogenität und in manchen Fällen zur Bildung von Trübungen, Ausfällungen und Bodensatz neigen und die haaravivierende Wirkung durch Interaktion des kationischen Polymeren mit der Seife allmählich verlorengeht.

Da Oxidationshaarfärbemittel-Zubereitungen auf Basis flüssiger, wässriger oder wässrig-alkoholischer Seifenlösungen andererseits durchaus erwünscht sind, da sie nach Zugabe der wässrigen Lösung des Oxidationsmittels eine dickflüssige bis gelförmige Färbezubereitung ausbilden, die bei der Anwendung am Haar haftet, bestand die Aufgabe, eine geeignete Formulierung aufzufinden, die es ermöglicht, Seifen und kationische Polymere in solche Haarfärbemittel-Grundlagen einzuarbeiten.

Es wurde gefunden, daß flüssige Zubereitungen für Oxidationshaarfärbemittel, bestehend aus Haarfarbstoff-Vorprodukten und einem wässrigen oder wässrig-alkoholischen Träger, mit einem Gehalt von (A) 5-20 Gew.-% Fettsäuren und 12-22 C-Atomen in Form einer wasserlöslichen Seife und (B) 0,5-10 Gew.-% eines wasserlöslichen kationischen Polymeren mit einem Molekulargewicht von 1000-3000000 dann homogen und lagerstabil bleiben, wenn sie 5-30 Gew.-% einer oder mehrerer Verbindungen aus der Gruppe der

(C) aliphatischen oder alicyclischen Dicarbonsäuren mit 10-44 C-Atomen in Form wasserlöslicher Salze und/oder

(D) Amine der allgemeinen Formel I

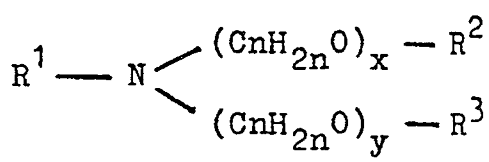

I

in der $R^1$ eine Alkylgruppe mit 8-22 C-Atomen, $R^2$ und $R^3$ unabhängig voneinander Wasserstoff oder eine Acylgruppe der Formel $R^4$-COO- bedeutet, in der $R^4$ eine Alkyl- oder Alkenylgruppe mit 1-21 C-Atomen ist, $n=2$ oder 3 und x und $y = 0$ oder Zahlen von 1 bis 5 sind, wobei die Summe $(x+y)$ 2 bis 6 betragen muß,

enthalten. Durch den Zusatz an Dicarbonsäure und/oder an Amin der vorgenannten Struktur wird das an sich instabile wässrige System aus Seife und kationischen Polymeren überraschend stabilisiert, und die avivierenden Eigenschaften des kationischen Polymeren bleiben erhalten.

Zur Bildung der Seifen (A) eignen sich bevorzugt Fettsäuren, die bei 20 °C flüssig sind, z.B. ungesättigte lineare Fettsäuren wie Ölsäure, Linolsäure, Palmitoleinsäure, Erucasäure oder flüssige Gemische dieser Fettsäuren untereinander und mit geringeren Anteilen gesättigter linearer Fettsäuren mit 12-22 C-Atomen. Andere bevorzugt geeignete flüssige Fettsäuren sind verzweigte Fettsäuren, z.B. die 2-Hexyl-decansäure, die 2-Octyl-dodecansäure oder die Isostearinsäure.

Zur Überführung der Fettsäuren in wasserlösliche Seifen eignen sich Alkalihydroxide und Alkalicarbonate, Ammoniak sowie Mono-, Di- und Trialkanolamine mit 2-4 C-Atomen in der Alkanolgruppe. Bevorzugt geeignet ist Ölsäure in Form der Ammonium-, Mono-, Di- und Triethanolammoniumseife.

Als wasserlösliche kationische Polymere (B) kommen prinzipiell alle Polymeren im Molekulargewichtsbereich von 1000 bis 3000000 in Frage, die entweder in der Polymerkette freie oder alkylsubstituierte Aminogruppen oder quartäre Ammoniumgruppen enthalten oder an die Polymerkette direkt oder über Zwischenglieder gebundene primäre, sekundäre oder tertiäre Aminogruppen oder quartäre Ammoniumgruppen tragen. Diese Aminogruppen oder quartären Ammoniumgruppen können auch Glieder von 5- oder 6-gliedrigen Ringsystemen, z.B. dem Morpholin-, Piperidin-, Piperazin- oder Imidazol-Ringsystem sein. Zahlche Beispiele für solche wasserlöslichen kationischen Polymeren sind z.B. in DE-OS 2.811.010 näher beschrieben. Darüberhinaus sind zahlreiche weitere wasserlösliche kationische Polymere literaturbekannt.

Bevorzugt geeignet sind wasserlösliche Homo- oder Mischpolymerisate (B1) mit Einheiten der allgemeinen Formel

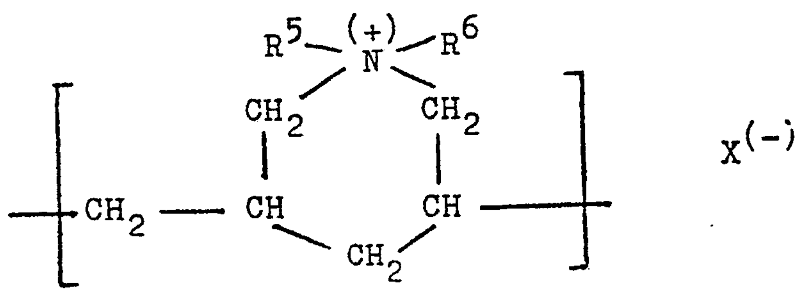

in der $R^5$ und $R^6$ Wasserstoff oder Alkylgruppen mit 1-4 C-Atomen oder Hydroxyalkylgruppen mit 2-4 C-Atomen sind und $X^{(-)}$ ein Chlorid, Bromid, Hydrogensulfat, Methosulfat-, Phosphat- oder Acetat-Anion ist. Beispiele für kationische Polymere dieser Art sind z.B. die Handelsprodukte Merquat® 100 und Merquat® 550 (Quaternium 41). Die Herstellung dieser Polymeren ist z.B. aus DE-OS 2.109.081 bekannt.

Weitere bevorzugt geeignete kationische Polymere sind Celluloseether (B2), deren Anhydroglucoseeinheiten 1-3 über Ethersauerstoff gebundene Substituenten mit quartären Ammoniumgruppen tragen. Solche Polymeren sind z.B. aus DE-OS 1.593.657 bekannt. Ein Handelsprodukt dieser Struktur ist z.B. Polymer JR® 400.

Bevorzugt geeignet sind auch die quartären polymeren Harnstoffderivate (B3), wie sie aus US-PS 4.157.388 bekannt sind. Ein Handelsprodukt dieses Typs ist Mirapol® A15, welches aus Struktureinheiten der allgemeinen Formel

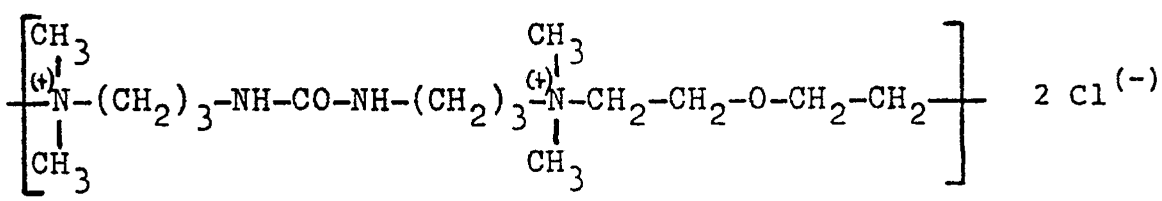

besteht, wobei der mittlere Polymerisationsgrad etwa 6 ist.

Als Dicarbonsäuren (C) sind lineare oder verzweigte, gesättigte oder ungesättigte Dicarbonsäuren mit 9-44 C-Atomen, z.B. Azelainsäure, Sebacinsäure, Brarsylsäure, Phellogensäure, die 2-Alkyl- und 2-Alkenylbernsteinsäuren mit 5-40 C-Atomen in der Alkyl- oder Alkenylgruppe geeignet. Die Alkyl- und Alkenylbernsteinsäuren sind literaturbekannte Verbindungen. Die Herstellung der Alkenylbernsteinsäureanhydride aus Maleinsäureanhydrid und Monoolefinen ist z.B. aus US-PS 2.411.215 bekannt. Die Alkenylbernsteinsäureanhydride können durch Hydrierung der Doppelbindung leicht in die entsprechenden Alkylbernsteinsäureanhydride überführt werden.

Andere geeignete Dicarbonsäuren sind die sogenannten Dimerfettsäuren, die durch thermische Dimerisation einfachund mehrfach ungesättigter Fettsäuren (z.B. von Mischungen aus Ölsäure und Linolsäure) im Sinne einer Diels-Alder-Addition zugänglich sind. Diese Dimerfettsäuren und ihre kommerzielle Herstellung sind z.B. in J. Am. Oil Chem. Soc. 39 (1962), S. 534 ff. näher beschrieben. Die zunächst entstehenden einfach ungesättigten Dimerfettsäuren lassen sich durch katalytische Hydrierung der Doppelbindung leicht in entsprechende gesättigte Dicarbonsäuren überführen. Solche Dimerfettsäuren sind auch im Handel erhältlich, z.B. unter dem Handelsnamen Empol® 1010 (Unilever Emery).

Auch eignen sich die En-Addukte von einfach ungesättigten Carbonsäuren wie Acryl- oder Methacrylsäure an ungesättigte Fettsäuren wie Undecylensäure, Ölsäure, Palmitoleinsäure, Linolsäure oder Erucasäure.

Eine bevorzugt geeignete verzweigte Dicarbonsäure dieses Typs ist die 5(6)-Carboxy-4-hexyl-2-cyclohexan-1-octansäure. Diese Dicarbonsäure wird in J. Am. Oil Chem. Soc. 52 (1975), S. 219-224 näher beschrieben und ist unter dem Handelsnamen Westvaco-Diacid 1550 im Handel erhältlich.

Die erfindungsgemäß enthaltenen aliphatischen und/oder alicyclischen Dicarbonsäuren liegen in den Haarfärbemittel-Zubereitungen in Form ihrer wasserlöslichen Salze vor; dies sind im allgemeinen Lithium-, Natrium-, Kalium-, Ammonium-, Mono-, Di- und/oder Triethanolammoniumsalze oder auch Isopropanolammoniumsalze. Es können aber auch Salze dieser Dicarbonsäuren mit anderen, anorganischen oder organischen Basen eingesetzt werden, vorausgesetzt, daß in den erfindungsgemäßen wässrigen oder wässrig-alkoholischen Haarfärbemittel-Zubereitungen wenigstens 5 Gew.-% der Dicarbonsäure in Form des Salzes gelöst ist.

Amine der allgemeinen Formel I

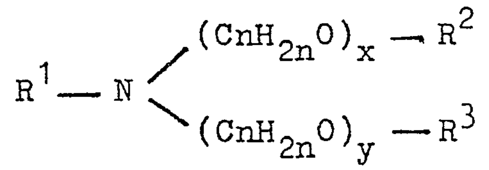

I

in der $R^1$, $R^2$, $R^3$, n, x und y die vorher genannte Bedeutung haben, sind aus primären Fettaminen mit 8-22 C-Atomen durch Anlagerung von (x+y)Mol Ethylenoxid oder Propylenoxid z.B. nach DE-PS 552.268 zugänglich. Durch Anlagerung von Ehtylenoxid oder Propylenoxid an Fettamine werden zunächst alkoxylierte Fettamine der allgemeinen Formel I erhalten, in der $R^2$ und $R^3$ Wasserstoff bedeuten. Diese Produkte lassen sich durch Veresterung mit Carbonsäuren der allgemeinen Formel $R^4$-COOH, in der $R^4$ eine Alkylgruppe mit 1-21 C-Atomen ist, oder mit Methylestern oder Säurechloriden dieser Carbonsäuren in die Produkte der allgemeinen Formel I überführen, bei welchen $R^2$ und $R^3$ eine Acylgruppe der Formel $R^4$-COO- darstellt. Zahlreiche Produkte der allgemeinen Formel I sind im Handel erhältlich. Ein Anlagerungsprodukt von 3 Mol Ethylenoxid an einen $C_{12}$-$C_{14}$-Fettalkohol wird z.B. unter der Bezeichnung Lowenol® C-243 verkauft. Ein Bis-(2-hydroxyethyl)-sojaalkylamindioleat ist unter der Bezeichnung Lowenol S-216 erhältlich. Weitere Fettamin-Oxalkylate sind unter der Bezeichnung Araphen®, Genamin®, Marlazin® oder Lutensol® im Handel.

Die erfindungsgemäßen Haarfärbemittel-Zubereitungen enthalten neben den genannten Trägerkomponenten Oxidationshaarfarbstoff-Vorprodukte. Als solche werden die bekannten Farbbasen bzw. Entwicklerverbindungen und bekannte Modifikatoren bzw. Kupplerverbindungen eingesetzt. Die Oxidationsfarbstoffe bilden sich durch oxidative Kupplung einer oder mehrerer Entwicklerkomponenten untereinander oder mit einer oder mehreren Kupplerkomponenten in Gegenwart eines Oxidationsmittels aus. Als Entwicklersubstanzen werden üblicherweise primäre aromatische Amine mit einer weiteren in Para- oder Orthoposition befindlichen freien oder substituierten Hydroxy- oder Aminogruppe, Diaminopyridin-Derivate, heterocyclische Hydrazonderivate, 4-Aminopyrazolonderivate oder Tetraaminopyrimidine eingesetzt. Als sogenannte Kupplersubstanzen werden z.B. m-Phenylendiaminderivate, Phenole, Naphthole, Resorcinderivate oder Pyrazolone verwendet. Die erfindungsgemäßen Haarfärbemittel-Zubereitungen können solche Oxidationshaarfarbstoff-Vorprodukte in einer Menge von 0,05-5,0 Gew.-%, bevorzugt von 0,2-2,0 Gew.-% enthalten.

Als weitere Hilfsmittel können die erfindungsgemäßen Haarfärbemittel-Zubereitungen noch synthetische anionische, nicht-ionogene, ampholytische oder zwitterionische Waschrohstoffe in Mengen bis zu 20 Gew.-% enthalten. Geeignet sind z.B. lineare Alkylsulfate mit 12-18 C-Atomen in der Alkylgruppe, Alkylpolyglycolethersulfate mit 12-16 C-Atomen in der Alkylgruppe und 1-6 Glycolethergruppen im Molekül, Fettalkoholpolyglycolether, die durch Anlagerung von 6-20 Mol Ethylenoxid an $C_{10}$-$C_{18}$-Fettalkohole erhalten werden, Anlagerungsprodukte von 6-20 Mol Ethylenoxid an Nonyl- oder Dodecylphenol, Fettalkyl-dimethylaminoxide, Fettsäure-mono-oder -diethanolamide, N-Fettalkyl-dimethyl-glycin, N-Fettalkylaminopropionsäure und andere bekannte oberflächenaktive Stoffe.

Außerdem können die erfindungsgemäßen Haarfärbemittel-Zubereitungen 0-20 Gew.-% Fettalkohole mit 12-22 C-Atomen, z.B. Kokosfettalkohol, Laurylalkohol, Myristylalkohol, Cetylalkohol, Oleylalkohol oder Stearylalkohol in emulgierter Form enthalten. Geeignet sind auch synthetische, verzweigte Alkohole, z.B. 2-Ocyldodecanol, 2-Hexyl-decanol, Isostearylalkohol, Isohexadecylalkohol.

Bevorzugt enthalten die erfindungsgemäßen Haarfärbemittel-Zubereitungen niedere Alkohole mit 1-4 C-Atomen und/oder niedere Glycole mit 2-6 C-Atomen, z.B. Ethanol, Isopropanol,n-Propanol, Ethylenglycol, 1,2-Propylenglycol, Methylglycol, Ethylglycol, Butylglycol, Diethylenglycol, Dipropylenglycol oder Hexylenglycol. Diese niederen Alkohole oder Glycole sind bevorzugt in einer Menge von insgesamt 10-30 Gew.-% in den Zubereitungen enthalten. Durch den Zusatz dieser niederen Alkohole und/oder Polyole bleiben die

Zubereitungen bei 20° C dünnflüssig und leicht verarbeitbar. Bei Zugabe einer etwa gleichen Menge Wasser oder wässriger Wasserstoffperoxidlösung, wie dies zur Entwicklung der Färbung kurz vor der Anwendung auf dem Haar erfolgt, bildet sich dann ein dickflüssiges bis gelförmiges gebrauchsfertiges Haarfärbepräparat.

Neben den bisher genannten Komponenten enthalten die erfindungsgemäßen Haarfärbemittel-Zubereitungen noch die in solchen Oxidationsfarbstoff-Grundlagen üblichen Zusätze zur Stabilisierung der Oxidationsfarbstoff-Vorprodukte; dies sind Komplexbildner, z.B. Ethylendiaminotetraessigsäure, Nitrilotriessigsäure, 1-Hydroxyethan-1.1-diphosphonsäure oder andere Organodiphosphonsäuren in Form ihrer Alkalisalze, Antioxidantien, wie z.B. Natriumsulfit, Natriumbisulfit, Hydrochinon oder Salze der Thioglycolsäure oder Ascorbinsäure, Puffersalze, wie z.B. Ammoniumsulfat, Ammoniumcarbonat, Ammoniumcitrat sowie Ammoniak oder ein Alkanolamin zur Einstellung eines pH-Wertes von 8-10.

Die folgenden Beispiele sollen den Gegenstand der Erfindung näher erläutern, ohne ihn hierauf zu beschränken:

## Beispiele

Zur Herstellung der Beispiele wurden folgende Produkte verwendet:

1) Farbstofflösung:

| | |
|---|---|
| Resorcin | 0,0825 g |
| p-Toluylendiamin | 0,2607 g |
| p-Aminophenol | 0,0662 g |
| 4-Chlorresorcin | 0,0545 g |
| 1,3-Bis(2.4-diaminophenoxy)-propan-tetrahydrochlorid | 0,0038 g |
| 2,4-Dichlor-3-aminophenol | 0,0470 g |
| Ammoniak (konz., ca. 25 Gew.-% in Wasser) | 0,4 g |
| Wasser | ad 7,0 g |

2) Stabilisatorlösung:

| | |
|---|---|
| Ethylendiamin-tetraessigsäure, Na-Salz | 0,2 g |
| $(NH_4)_2SO_4$ | 1,0 g |
| $Na_2SO_3$ | 0,3 g |
| Na-Ascorbat | 0,2 g |
| Ammoniak (konz., ca. 25 Gew.-% in Wasser) | 0,1 g |
| Wasser | ad 8,0 g |

3) Mirapol® A-15: Poly [N- [3(dimethyl(ammonio)propyl] - N'-[3-(ethylen-oxyethylendimethyl-ammonio)propyl-] harnstoff-dichlorid]

4) Lowenol C243: Addukt von 3 Mol Ethylenoxid an Kokosalkylamin

5) LowenolS216X: Bis(2-hydroxyethyl)sojaalkylamin-dioleat

6) Polymer JR400: Kationisches Cellulosederivat

Oxidationshaarfärbemittel-Zubereitungen

Beispiel 1

| | |
|---|---|
| Ammoniumoleat (85 Gew.-% in Wasser) | 7,5 g |
| Fettalkohol $C_{12}$-$C_{14}$-2EO-sufat, Na-Salz (28 Gew.-% in Wasser, Texapon® N25) | 3,5 g |
| Nonylphenolpolyglycolether (9EO) | 12,0 g |
| Laurylalkohol | 6,5 g |
| 1,2-Propylenglycol | 8,0 g |
| Wasser | 15,0 g |
| Stabilisatorlösung[2)] | 8,0 g |
| Farbstofflösung[1)] | 7,0 g |
| Mirapol® A15 [3)] | 3,0 g |
| Westvaco Diacid 1550 | 10,0 g |
| Parfümöl | 0,2 g |
| Ammoniak (konz., ca. 25 Gew.-% in Wasser) | 5,0 g |
| Isopropanol | 10,0 g |
| Wasser | ad 100,0 g |

Herstellung: Ammoniumoleat, Fettalkoholethersulfat, Nonylphenol-polyglycolether, Laurylalkohol und 1,2-Propylenglycol wurden mit 15 g Wasser auf 35 °C erwärmt, die Stabilisatorlösung, die Farbstofflösung, das Gemisch aus Mirapol® A15 und Westvaco Diacid® 1550 und der Duftstoff wurden dann der Reihe nach zugegeben, mit Ammoniaklösung der pH-Wert auf 10 eingestellt, dann Isopropanol und das restliche Wasser zugesetzt.

Beispiel 2

| | |
|---|---|
| Propylenglycol | 8,6 g |
| Lowenol® C243[4)] | 8,0 g |
| Isopropanol | 12,5 g |
| Ölsäure | 8,6 g |
| LowenolS-216-X [5)] | 21,6 g |
| Monoethanolamin | 8,0 g |
| Stabilisatorlösung [2)] | 8,0 g |
| Farbstofflösung [1)] | 7,0 g |
| Polymer JR® 400 [6)] | 2,0 g |
| Wasser | 23,0 g |
| Parfümöl | 0,2 g |
| Wasser | ad 100,0 g |

Herstellung: Propylenglycol und Lowenol® C243 wurden gemeinsam auf 70 °C erwärmt, Isopropanol zugegeben und weiter bei 70 °C verrührt, bis eine klare Lösung gebildet war. Dann wurde Ölsäure, Lowenol® S216X und Monoethanol zugesetzt. Nach dem Abkühlen wurden die Stabilistorlösung, die Farbstofflösung, eine Lösung von Polymer JR® 400 in 23 g Wasser, das Parfümöl und die Restmenge an Wasser der Reihe nach zugegeben und untergerührt. Der pH-Wert wurde mit Monoethanolamin auf 10,0 eingestellt.

Die Haarfärbemittel-Zubereitungen gemäß Beispiel 1 und 2 sind niedrigviskose Flüssigkeiten. Kurz vor der Anwendung wurden sie mit 1 Gewichtsteil 6 Gew.-%iger Wasserstoffperoxid-Lösung pro 1 Gewichtsteil Zubereitung vermischt. Dabei wurden dickflüssige bis gelförmige Färbeprodukte erhalten.

## Ansprüche

1. Flüssige Zubereitung für Oxidationshaarfärbemittel, bestehend aus Haarfarbstoff-Vorprodukten und einem wässrigen oder wässrig-alkoholischen Träger mit einem Gehalt von (A) 5-30 Gew.-% Fettsäuren mit 12-22 C-Atomen in Form einer wasserlöslichen Seife und (B) 0,5-10 Gew.-% eines wasserlöslichen kationischen Polymeren mit einem Molekulargewicht von 1000-3000000, dadurch gekennzeichnet, daß zur Stabilisierung ein Zusatz von 5-30 Gew.-% einer oder mehrerer Verbindungen aus der Gruppe der

   (C) aliphatischen oder alicyclischen Dicarbonsäuren mit 9-44 C-Atomen in Form wasserlöslicher Salze und/oder der

   (D) Amine der allgemeinen Formel

   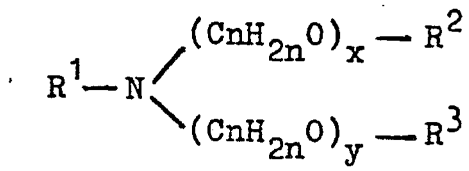

   in der $R^1$ eine Alkylgruppe mit 8-22 C-Atomen, $R^2$ und $R^3$ unabhängig voneinander Wasserstoff oder eine Acylgruppe der Formel $R^4$-COO- bedeutet, wobei $R^4$ eine Alkylgruppe mit 1-21 C-Atomen ist, n = 2 oder 3 und x und y = 0 oder Zahlen von 1 bis 5 sind, wobei die Summe (x+y) 2 bis 6 betragen muß,

   enthalten sind.

2. Flüssige Zubereitung nach Anspruch 1, dadurch gekennzeichnet, daß als Fettsäure (A) Ölsäure in Form von Ammonium-, Mono-, Di- oder Triethanolammoniumoleatund als wasserlösliches kationisches Polymeres (B) eine polymere quartäre Ammoniumverbindung aus der Gruppe der

   (B1) wasserlöslichen Homo- und Mischpolymerisate mit Einheiten der allgemeinen Formel

   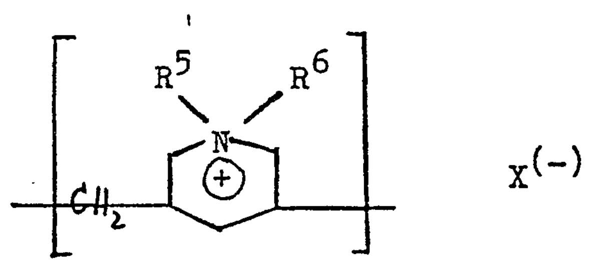

   in der $R^5$ und $R^6$ Alkylgruppen mit 1-4 C-Atomen oder Hydroxyalkylgruppen mit 2-4 C-Atomen sind und $x^{(-)}$ ein Chlorid-, Bromid-, Hydrogensulfat-, Methosulfat-, Phosphat- oder Acetat-Anion ist,

   (B2) Celluloseether, deren Anhydroglucoseeinheiten 1-3 über Ethersauerstoff gebundene Substituenten mit quartären Ammoniumgruppen tragen;

   (B3) polymeren, quartären Harnstoffderivaten mit Einheiten der allgemeinen Formel

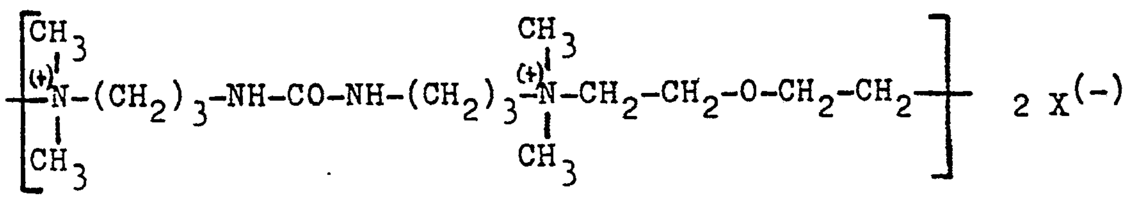

in der $X^{(-)}$ die vorgenannte Bedeutung hat, enthalten ist.

3. Flüssige Zubereitung nach Anspruch 1 oder 2, dadurch gekennzeichnet, daß als Dicarbonsäure eine 5-(6)-Carboxy-4-hexyl-2-cyclohexan-1-octansäure enthalten ist.

4. Flüssige Zubereitungen nach Anspruch 1 bis 3, dadurch gekennzeichnet, daß niedere Alkohole mit 1-4 C-Atomen und/oder Glycole mit 2-6 C-Atomen in einer Menge von insgesamt 10-30 Gew.-% darin enthalten sind.

## Claims

1. A liquid preparation for oxidation hair dyes consisting of hair dye precursors and an aqueous or aqueous-alcoholic carrier containing (A) from 5 to 30% by weight of $C_{12}$-$C_{22}$ fatty acids in the form of a water-soluble soap and (B) from 0.5 to 10% by weight of a water-soluble cationic polymer having a molecular weight of from 1,000 to 3,000,000 characterized in that it contains for stabilization an addition of from 5 to 30% by weight of one or more compounds from the group comprising
(C) aliphatic or alicyclic $C_9$-$c_{44}$ dicarboxylic acids in the form of water-soluble salts and/or
(D) amines corresponding to the following general formula

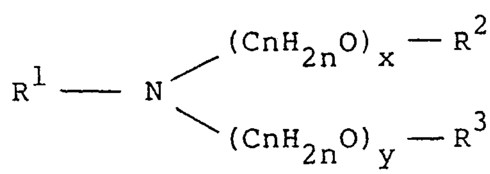

in which $R^1$ is a $C_8$-$C_{22}$ alkyl group, $R^2$ and $R^3$ independently of one another represent hydrogen or an acyl group of the formula $R^4$-COO- where $R^4$ is a $C_1$-$C_{21}$ alkyl group, n = 2 or 3 and x and y = 0 or numbers of from 1 to 5, x and y together making a total of 2 to 6.

2. A liquid preparation as claimed in claim 1, characterized in that it contains oleic acid in the form of ammonium, mono-, di- or triethanolammonium oleate as the fatty acid (A) and, as the water-soluble cationic polymer (B), a polymeric quaternary ammonium compound from the group comprising
(B1) water-soluble homopolymers and copolymers containing units corresponding to the following general formula

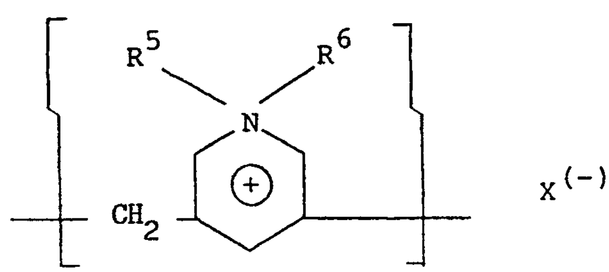

in which $R^5$ and $R^6$ are $C_1$-$C_4$ alkyl groups or $C_2$-$C_4$ hydroxyalkyl groups and $X^{(-)}$ is a chloride,

bromide, hydrogen sulfate, methosulfate, phosphate or acetate anion,

(B2) cellulose ethers of which the anhydroglucose units bear from 1 to 3 substituents containing quaternary ammonium groups attached by ether oxygen;

(B3) polymeric, quaternary urea derivatives containing units corresponding to the following general formula

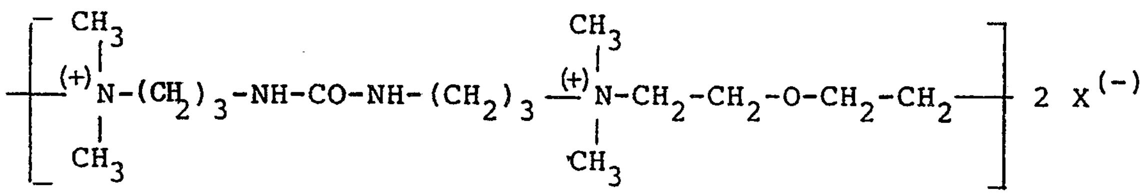

in which $X^{(-)}$ is as defined above.

3. A liquid preparation as claimed in claim 1 or 2, characterized in that it contains a 5(6)-carboxy-4-hexyl-2-cyclohexane-1-octanoic acid as the dicarboxylic acid.

4. Liquid preparations as claimed in claims 1 to 3, characterized in that they contain lower $C_1$-$C_4$ alcohols and/or $c_2$-$C_6$ glycols in a total quantity of from 10 to 30% by weight.

**Revendications**

1. Préparation liquide pour les teintures pour cheveux par oxydation, se composant de précurseurs de colorants pour cheveux et d'un support aqueux ou aqueux-alcoolique, avec une concentration de (A) 5 à 30 % en poids d'acides gras et de 12 à 22 atomes de carbone sous la forme d'un savon soluble dans l'eau et de (B) 0,5 à 10 % en poids d'un polymère cationique soluble dans l'eau dont le poids moléculaire est compris entre 1 000 et 3 000 000, caractérisée en ce qu'elle contient pour la stabiliser un supplément de 5 à 30 % en poids d'un ou de plusieurs composés faisant partie du groupe des

(C) acides dicarboxyliques aliphatiques ou alicycliques comportant 9 à 44 atomes de carbone sous la forme de sels solubles dans l'eau et/ou des

(D) amines de la formule générale I

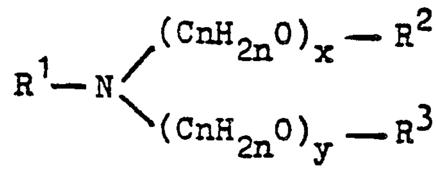

dans laquelle $R^1$ représente un groupement alkyle comportant 8 à 22 atomes de C, $R^2$ et $R^3$, peuvent être indépendamment l'un de l'autre l'hydrogène ou un groupement acyle de la formule $R^4COO$-, dans laquelle $R^4$ représente un groupe alkyle comportant 1 à 21 atomes de C, n est égal à 2 ou à 3 et x et y sont égaux à 0 ou à des nombres de 1 à 5, la somme (x + y) devant atteindre 2 à 6.

2. Préparation fluide selon la revendication 1, caractérisée en ce qu'elle contient comme acide gras (A), de l'acide oléique sous la forme d'oléate d'ammonium, de mono-, de di- ou de triéthanolammonium et comme polymère cationique soluble dans l'eau (B), un composé d'ammonium polymère quaternaire faisant partie du groupe des

(B1) homo- et copolymères solubles dans l'eau comportant des unités de la formule générale

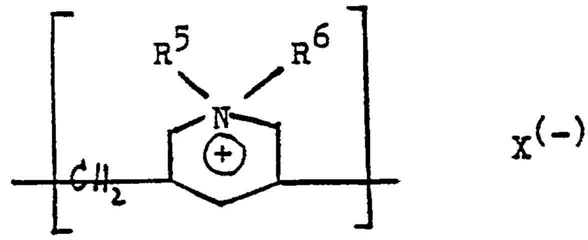

dans laquelle $R^5$ et $R^6$ représentent des groupements alkyle comportant 1 à 4 atomes de carbone ou des groupements hydroxyalkyle comportant 2 à 4 atomes de carbone et $X^{(-)}$est un anion chlorure, bromure, hydrogénosulfate, méthosulfate, phosphate ou acétate,

(B2) des éthers cellulosiques dont les unités anhydroglucosiques 1 à 3 portent des substituants fixés par l'intermédiaire d'oxygène d'éther avec des groupements d'ammonium quaternaires,

(B3) des dérivés d'urée polymères quaternaires comportant des unités de la formule générale

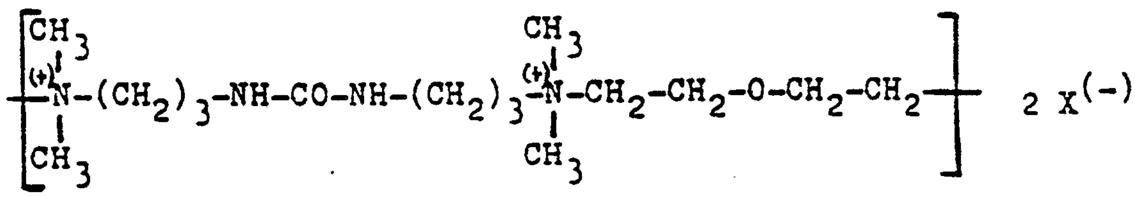

dans laquelle $X^{(-)}$possède la signification précitée.

**3.** Préparation fluide selon la revendication 1 ou 2, caractérisée en ce qu'elle contient comme acide dicarboxylique, un acide 5(6)-carboxy-4-hexyl-2-cyclohexane-1-octanoïque.

**4.** Préparation fluide selon les revendications 1 à 3, caractérisée en ce qu'elle contient des alcools inférieurs comportant 1 à 4 atomes de carbone et/ou des glycols inférieurs comportant 2 à 6 atomes de carbone dans une proportion totale de 10 à 30 % en poids.